# EUROPÄISCHE PATENTSCHRIFT

(11) **EP 1 292 383 B1**
(45) Veröffentlichungstag und Bekanntmachung des Hinweises auf die Patenterteilung: **04.08.2004**
(21) Anmeldenummer: 01933928.2
(22) Anmeldetag: 04.05.2001
(51) Int. Cl.: B01J 8/06

(54) **RÖHRENREAKTOR ZUR DURCHFÜHRUNG EXOTHERMER GASPHASENREAKTIONEN**
TUBULAR REACTOR FOR CARRYING OUT EXOTHERMIC GAS PHASE REACTIONS
REACTEUR TUBULAIRE UTILISE POUR DES REACTIONS EXOTHERMIQUES EN PHASE GAZEUSE

(30) Priorität: 05.05.2000 DE 10021986
(43) Veröffentlichungstag der Anmeldung: 19.03.2003
(73) Patentinhaber: MAN DWE GmbH, 94469 Deggendorf (DE)
(72) Erfinder: GÜTLHUBER, Friedrich, 94526 Metten (DE)
(74) Vertreter: Lieck, Hans-Peter, Dipl.-Ing.
(86) Internationale Anmeldenummer: PCT/EP2001/005030
(87) Internationale Veröffentlichungsnummer: WO 2001/085332

(56) Entgegenhaltungen:
- WO-A-97/05947
- WO-A-98/55216
- DE-A- 1 667 247
- US-A- 3 268 299
- US-A- 3 518 284
- PATENT ABSTRACTS OF JAPAN vol. 012, no. 372 (C-533), 5. Oktober 1988 (1988-10-05) -& JP 63 123433 A (MITSUBISHI HEAVY IND LTD;OTHERS: 01), 27. Mai 1988 (1988-05-27)

## Beschreibung

Die Erfindung betrifft einen Röhrenreaktor gemäß Gattungsbegriff des Patentanspruchs 1.

Röhrenreaktoren finden für vielerlei chemische Reaktionsprozesse Verwendung, unter anderem die katalytische Oxidation von Kohlenwasserstoffen wie zum Beispiel zur Herstellung von Ethylenoxid oder von Essigsäure. Dabei erfolgt die Umsetzung beispielsweise im Kreislaufverfahren, wobei vor Eintritt in den Reaktor laufend frisches Reaktionsgas zugesetzt und nach dem Austritt aus dem Reaktor die abzuführenden Stoffströme abgetrennt werden. Die Ausbeute pro Durchgang und damit auch die Größe des Reaktors samt zugehöriger Aggregate wie Pumpen, Gebläse und dergl. sowie die dafür erforderliche Antriebsleistung hängen wesentlich von der Effizienz der Umsetzung und diese wiederum von dem Mischungsverhältnis der Reaktanten ab. Dieses findet indessen Grenzen in der Beherrschbarkeit der anfallenden Reaktionswärmemenge und in manchen Fällen auch in einem Abbrand- oder sogar Explosionsrisiko. So ist man dabei herkömmlicherweise etwa gezwungen, den O₂-Anteil am Eintritt des Reaktors auf wenige Prozent zu begrenzen. Ähnliche Probleme bestehen beispielsweise bei der Herstellung von Phthalsäureanhydrid, Maleinsäureanhydrid, Acrolein und Acrylsäure.

Durch mancherlei Maßnahmen ist es bereits gelungen, die Beladung des Reaktionsgasgemisches mit einer kritischen Komponente, wie z.B. O₂, dadurch zu steigern, daß man die sich entlang den Kontaktrohren einstellende Temperatur in gewünschter Weise über den Wärmeträgerkreislauf steuert. Maßnahmen hierfür sind etwa DE-C-2 201 528 zu entnehmen, wonach der Reaktor in mehrere aufeinanderfolgende Abschnitte mit mehr oder weniger separaten Wärmeträgerkreisläufen und ggf. auch unterschiedlichen Katalysatorfüllungen unterteilt sein kann und dazu noch verschiedenartige Umlenk- und/oder Verteilerplatten Verwendung finden können. Des weiteren wurde bereits vorgeschlagen, zumindest einen Anteil der kritischen Komponente erst nach und nach im Zuge des Reaktionsablaufes zuzuführen, so z.B. mittels durch die Reaktionsrohre hindurchlaufender separater Gaszuführungsrohre mit verstreuten oder auch diskreten aufeinanderfolgenden Gasaustrittsstellen (Tonkovich et al. "Inorganic Membrane Reactors for the Oxidative Coupling of Ethane", Chem. Engineering Science, Vol. 51, No. 11, 1996, S. 3051 - 3056, bzw. US-A-5,723,094). Derartige Gaszuführungsrohre sind freilich, vor allem in industriellem Umfang, nur schwer zu verwirklichen, nicht zuletzt was eine wünschenswerte Verteilung des Gasaustritts über die Rohrlänge angeht, aber auch hinsichtlich der Gaszuführung zu einer Vielzahl solcher Gaszuführungsrohre, die bei einem industriellen Röhrenreaktor etwa 10.000 oder mehr betragen kann.

Aus US-A-3,518,284 - wovon im Gattungsbegriff des Patentanspruchs 1 ausgegangen wird - ist des weiteren die Zuführung eines zweiten Reaktanten durch lediglich in das gaseintrittsseitige Ende der Reaktionsrohre hineinragende separate Gaszuführungsrohre bekannt, die noch vor der Katalysatorfüllung, ggf. in einer vorgelagerten Inertmaterialfüllung, enden können. Dabei geht man aus von der Erkenntnis, daß es genügt, die Verweilzeit des fertigen Reaktionsgasgemischs vor der bewußten Einleitung der Reaktion wie auch das für das fertige Reaktionsgasgemisch verfügbare Volumen möglichst klein zu halten, um das Explosionsrisiko auszuschalten. Den betreffenden separaten Gaszuführungsrohren wird der zweite Reaktant über eine Rohrverzweigung im Inneren der gaseintrittsseitigen Haube zugeführt, was jedoch der Anzahl der Reaktionsrohre Grenzen setzt. In einer Variante schlägt die betreffende Literaturstelle vor, den zweiten Reaktanten aus einer das Eintrittsende des Reaktionsrohrbündels in sich aufnehmenden zweiten Gaszuführungskammer über radiale Bohrungen der Reaktionsrohre hindurch zuzuführen.

Schließlich ist noch aus "Patent Abstracts of Japan, vol. 012, no. 372 (C-533) vom 5.10.1988 bzw. JP-63 123 433 A ein Röhrenreaktor mit zwei separaten Gaszuführungskammern innerhalb der gaseintrittsseitgen Haube bekannt, die durch einen regelrechten Rohrboden für weit in die Reakionsrohre hineinragende separate Gaszuführungsrohre voneinander getrennt sind.

Hier wie dort fällt es schwer, die über die einzelnen separaten Gaszuführungsrohre zugeführte Menge des zweiten Reaktanten dem jeweiligen Bedarf entsprechend im Verhältnis zu dem ersten Reaktanten genau zu dosieren.

Von daher liegt der Erfindung die Aufgabe zugrunde, bei einem Röhrenreaktor gemäß Gattungsbegriff des Patentanspruchs 1 die Möglichkeit zu schaffen, eine entflammungs- oder explosionskritische Reaktionskomponente derart zuzuführen, daß diese Komponente geeignet zudosiert werden kann, um die Reaktionswärmeenergie zu steuern und im Falle einer Fehlsteuerung die Zuführung dieser Komponente rasch zu unterbinden und den sich innerhalb des Reaktors einstellenden Druckanstieg gering und leicht beherrschbar zu halten.

Diese Aufgabe ist maßgeblich mit den Kennzeichnungsmerkmalen des Patentanspruchs 1 gelöst. Die Unteransprüche geben dazu vorteilhafte Ausgestaltungsmöglichkeiten oder auch Zusatzmaßnahmen an.

Die betreffenden separaten, kurzen Gaszuführungsrohre in Verbindung mit einer zugehörigen zweiten Gaszuführungskammer innerhalb der Gaseintrittshaube ermöglichen es, eine entflammungs- oder explosionskritische Reaktionsgaskomponente erst unmittelbar vor Beginn der angestrebten Reaktion zuzuführen und, sollte es - etwa infolge einer Fehlsteuerung - doch einmal zu einer Entflammung oder gar Explosion kommen, die Zufuhr dieser Komponente rasch zu unterbinden, vor allem wenn die betreffende Gaszuführungskammer ein kleines Volumen erhält, um so die Ausbreitung des Abbrands im Reaktor zu begrenzen und vor allem den sich innerhalb des Reaktors einstellenden Druckanstieg gering und leicht beherrschbar zu halten. Dazu noch erübrigen sich Abdichtungsprobleme bezüglich einer Wandhindurchführung der Gaszuführungsrohre, wie sie etwa nach US-A-5,723,094 zu erwarten sind. Auch können die Gaszufuhrungsrohre nach der Erfindung mitsamt ihrer Gaszuführungskammer entnommen werden, um so die Befüllung der Reaktionsrohre mit Katalysator nicht zu behindern.

Die individuellen Drosselorgane für die Gaszuführungsrohre lassen eine genaue Festlegung der durch sie zugeführten Menge des zweiten Reaktanten im Verhältnis zur Menge des ersten Reaktanten unabhängig vom Ort der zugehörigen Reaktionsrohre in bezug auf den Reaktorquerschnitt zu. Dazu noch verhindern die Drosselorgane eine Brandfortsetzung, sollte es doch einmal vor oder hinter dem jeweiligen Drosselorgan zu einem Brand kommen.

Nachfolgend werden einige vorteilhafte Ausführungsbeispiele und Ausgestaltungsmoglichkeiten der Erfindung anhand der Figuren genauer beschrieben. Von diesen zeigt
- Fig. 1: einen schematischen Längsschnitt durch einen erfindungsgemäßen Röhrenreaktor gemäß einem ersten Ausführungsbeispiel,
- Fig. 2: einen schematischen Längsschnitt durch den Gaseintrittsbereich eines erfindungsgemäßen Röhrenreaktors in einer anderen Ausführungsform,
- Fig. 3: einen ebensolchen Längsschnitt gemäß einem dritten Ausführungsbeispiel,
- Fig. 4: einen ebensolchen Längsschnitt gemäß einem vierten Ausfuhrungsbeispiel,
- Fig. 5: einen ebensolchen Längsschnitt gemäß einem fünften Ausführungsbeipsiel,
- Fig. 6,: stark vergrößert, eine beispielhafte Ausbildung des Gaszutritts zu den Gaszuführungsrohren nach der Erfindung samt deren Anschluß an den betreffenden Rohrboden,
- Fig. 7: eine andere beispielhafte Ausführungsform des Gaszutritts zu den Gaszuführungsrohren,
- Fig. 8: eine dritte beispielhafte Ausführungsform des Gaszutritts zu den Gaszuführungsrohren,
- Fig. 9: noch eine weitere beispielhafte Ausfuhrungsform des Gaszutritts zu den Gaszufuhrungsrohren,
- Fig. 10: einen geschnittenen Abschnitt eines Reaktionsrohres mit dem darin befindlichen Ende des betreffenden Gaszufuhrungsrohres und
- Fig. 11: ein Schema eines erfindungsgemäßen Rohrenreaktors ähnlich demjenigen der Fig. 1, jedoch mit schematisch angegebenen Mitteln für eine besondere Art der Temparatursteuerung entlang den Kontaktrohren.

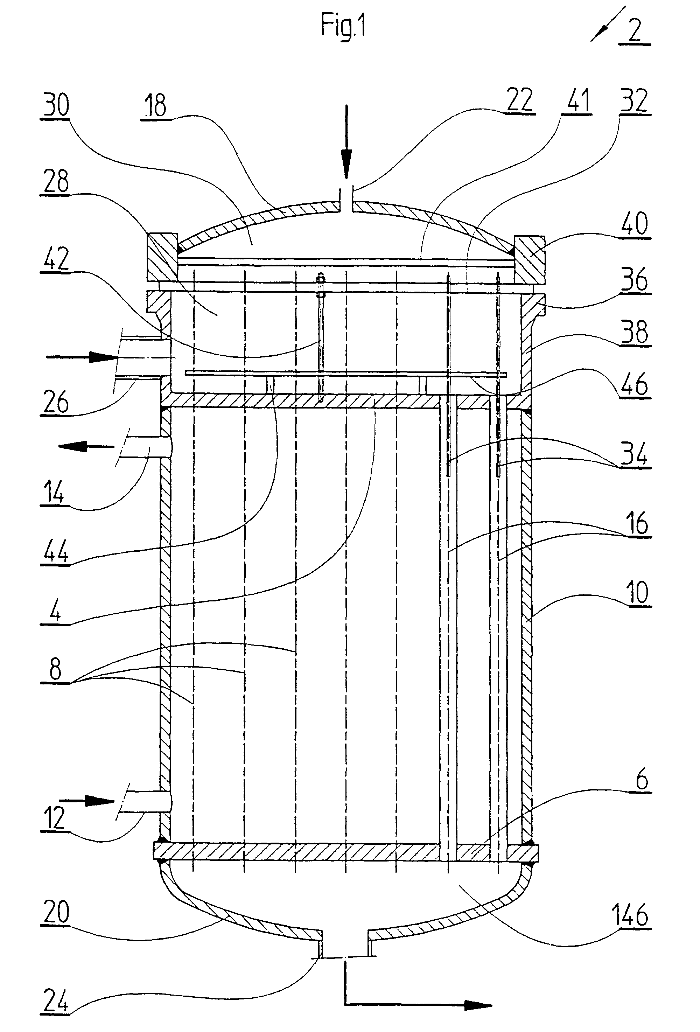

Der in Fig. 1 dargestellte Röhrenreaktor 2 besitzt in insoweit üblicher Weise ein sich abgedichtet zwischen zwei Rohrböden 4 und 6 erstreckendes Reaktionsrohrbündel 8, das innerhalb eines umgebenden Reaktormantels 10 von einem im Betrieb flussigen Warmetrager, gewohnlich in Gestalt eines Salzbades, umspült wird. In dem gezeigten Beispiel tritt Wärmeträger am gasaustrittsseitigen Ende des Reaktormantels 10 durch einen Rohrstutzen 12 ein und am gaseintrittsseitigen Ende durch einen Rohrstutzen 14 aus, jedoch könnten Ein- und Austritt des Warmeträgers auch in bekannter Weise über Ringkanäle erfolgen, ebenso wie der Warmeträger generell im Gleichstrom anstatt im Gegenstrom in bezug auf das Reaktionsgasgemisch durch den Reaktormantel 10 hindurchtreten konnte. Sodann könnte das Reaktionsgasgemisch auch von unten nach oben anstatt, wie gezeigt, von oben nach unten durch die Reaktionsrohre 16 hindurchtreten.

Den stirnseitigen Abschluß des Reaktors 2 bilden im wesentlichen nach außen gewölbte Hauben 18 und 20 mit zentralen Gaseintritts- und Gasaustrittsstutzen 22 bzw. 24.

Wahrend jedoch bei herkömmlichen Röhrenreaktoren die gaseintrittsseitige Haube selbst eine einzige mit den Reaktionsrohren 16 in Verbindung stehende Gaszuführungskammer bildet, um den Reaktionsrohren fertig vorgemischtes Reaktionsgas zuzuführen, ist bei dem Reaktor 2 gemäß Fig. 1 zwischen die gaseintrittsseitige Haube, 18, und den gaseintrittsseitigen Rohrboden 4 zur Speisung der Reaktionsrohre 16 eine durch einen seitlichen Rohrstutzen 26 hindurch speisbare erste Gaszufuhrungskammer 28 zwischengeschaltet, die von einer unter der Haube 18 liegenden zweiten Gaszuführungskammer 30 durch einen weiteren Rohrboden, 32, getrennt ist. In dem Rohrboden 32 sind, darin abgedichtet, nach unten hin bis in die Reaktionsrohre 16 hineinragende Gaszuführungsrohre 34 für die Einspeisung eines zweiten gasförmigen Reaktanten verankert, der in die zweite Gaszuführungskammer 30 durch den Gaseintrittsstutzen 22 hindurch eintritt. Der Rohrboden 32 ist abgedichtet zwischen einem Flansch 36 der zylindrischen Seitenwand 38 und einem entsprechenden Flansch 40 an der Haube 18 eingespannt. Im Gegensatz zu den Rohrböden 4 und 6, die das Gewicht des Rohrbündels 8 und zum Teil auch dasjenige des innerhalb des Reaktormantels 10 befindlichen Wärmetragers zu tragen sowie ggf. einer größeren Druckdifferenz zu widerstehen haben, kann der Rohrboden 32 bei geringer Druckdifferenz zwischen dem ersten und zweiten eingefuhrten Reaktanten verhaltnismäßig leicht ausgeführt werden. Dies gilt umsomehr, wenn der Rohrboden 32, wie gezeigt, auf dem Rohrboden 4 mittels Tragstangen 42 abgestützt ist.

Innerhalb der Gaszuführungskammer 30 ist eine transversale Gasverteilungsplatte 41 zu erkennen, die mit nach Strömungsverteilungsgesichtspunkten variierenden Durchbrechungen prinzipiell ähnlich gestaltet sein kann wie die in DE-C-2 201 528 (dort allerdings für den Warmeträger) angegebenen "Umlenkscheiben" 60 und 61.

Eine über Füße 44 mit einigem Abstand auf dem Rohrboden 4 aufliegende, vorzugsweise aber dennoch gasdurchlassige Zentrierplatte 46 hält die unteren Enden der Gaszuführungsrohre 34 in zentrierter Position in bezug auf die Reaktionsrohre 16. Bei großer Gasdurchlässigkeit, wie sie bei einer solchen Platte ohne weiteres zu verwirklichen ist, kann die Zentrierplatte 46 auch unmittelbar auf dem Rohrboden 4 aufliegen. Die Zentrierplatte 46 ist auf den Gaszuführungsrohren 34 verschiebbar. Nach Entfernen der Haube 18 kann der Rohrboden 32 mitsamt den Gaszuführungsrohren 34 und der Zentrierplatte 46 entnommen werden, welche dabei auf Vorsprüngen an den unteren Enden der Gaszuführungsrohre 34 zu liegen kommt, um so beim Wiederzusammenbau die Einführung der Gaszuführungsrohre 34 in die zugehörigen Reaktionsrohre 16 zu erleichtern.

Soweit bei den nachfolgend beschriebenen weiteren Ausführungsbeispielen gleichartige Elemente auftreten, finden für diese die gleichen Bezugszeichen Verwendung wie bei dem Ausführungsbeispiel nach Fig. 1.

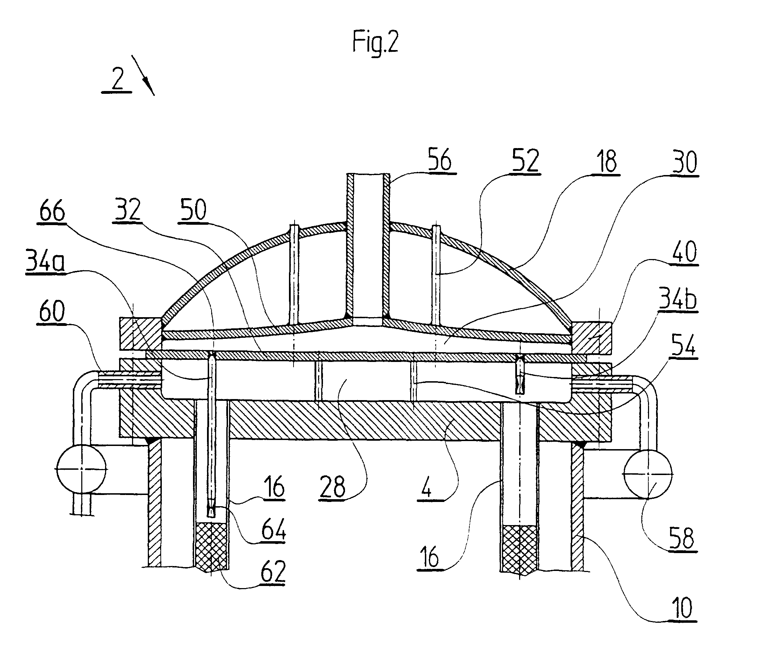

Gemäß Fig. 2 ist das Volumen der zweiten Gaszuführungskammer 30 von einem Einbau in Gestalt einer in die Haube 18 eingeschweißten Platte 50 begrenzt, die sich an der Haube 18 über Tragstangen 52 abstützt, ebenso wie der Rohrboden 32 sich an dem Rohrboden 4 über Tragstangen 54 abstützt. Während die Tragstangen 52 an die Haube 18 angeschweißt sind, ruhen die Tragstangen 54 aus Demontierbarkeitsgründen auf dem Rohrboden 4 lose auf.

In diesem Fall erfolgt die Zuführung des zweiten Reaktanten zu der zweiten Gaszuführungskammer 30 über ein massives, die Haube 18 durchsetzendes zentrales Rohr 56, das zugleich ebenfalls der Abstützung der Platte 50 an der Haube 18 dienen kann. Wie ersichtlich, ist die Platte 50 gewölbt, um auf diese Weise der Kammer 30 eine nach außen zu abnehmende Höhe zu vermitteln, welche der nach außen zu abnehmenden Menge des radial durch die Kammer 30 hindurchtretenden zweiten Reaktanten entspricht, um auf diese Weise der Kammer 30 ein kleinstmögliches Volumen zu verleihen. Als weitere Variante gegenuber Fig. 1 erfolgt die Zuführung des ersten Reaktanten zu der ersten Gaszuführungskammer 28 gemäß Fig. 2 aus einer Ringleitung 58 über eine Mehrzahl radial einmündender Rohre 60. Dies erlaubt es, auch die Gaszuführungskammer 28 niedrig und so ihr Volumen klein zu halten, um auch die Zuführ des ersten Reaktanten - gewöhnlich eines bereits für sich reaktionsfähigen Gemischs - rasch unterbinden und überdies seine Verweildauer in dem Reaktor gering halten zu können. Es hat sich nämlich gezeigt, daß die Selbstzündwahrscheinlichkeit eines insofern kritischen Gasgemisches mit der Dauer seines Bestehens zunimmt.

In bezug auf die Gaszuführungsrohre 34 sind auf beiden Seiten der Fig. 2 zwei Varianten gezeigt, die in Wirklichkeit alternativ und nicht nebeneinander auftreten werden. Auf der linken Seite ist ein Gaszuführungsrohr 34a in das betreffende Reaktionsrohr 14 hinein und bis unmittelbar vor eine darin befindliche Katalysatorfüllung 62 reichend gezeigt, wahrend das Gaszuführungsrohr 34b auf der rechten Seite der Fig. 2 nur bis vor das gaseintrittsseitige Ende des zugehörigen Reaktionsrohres 16 reicht. Statt frei vor der Katalysatorfüllung 62 könnte das Gaszuführungsrohr 34a freilich auch innerhalb einer dieser vorausgehenden Inertmaterialschicht enden.

In beiden gezeigten Fällen ist am Ende des Gaszuführungsrohres 34 eine Mischdüse 64, hier in Gestalt eines Venturirohres, zu erkennen, während sich am Eintrittsende des Gaszuführungsrohres ein Drosselorgan 66 befindet, um den Gaszutritt zu wie auch den Gasaustritt aus dem Gaszuführungsrohr 34 zu dosieren. Diese Dosierung vermag erforderlichenfalls auch einem radialen Druckabfall innerhalb der Gaszuführungskammer 30 Rechnung zu tragen. Die Mischdüse 64 soll eine möglichst rasche und effektive Beimischung des zweiten zu dem ersten Reaktanten bewirken. Unter Umständen können an dem Gaszuführungsrohr (34) auch mehrere solche Mischdüsen vorgesehen sein.

Es versteht sich, daß der betreffende Reaktor 2 in Wirklichkeit weit mehr als die gezeigten zwei Reaktionsrohre 16 und Gaszuführungsrohre 34 aufweisen wird und die Darstellung in Fig. 2 (und weiteren) nur illustrativ zu verstehen ist.

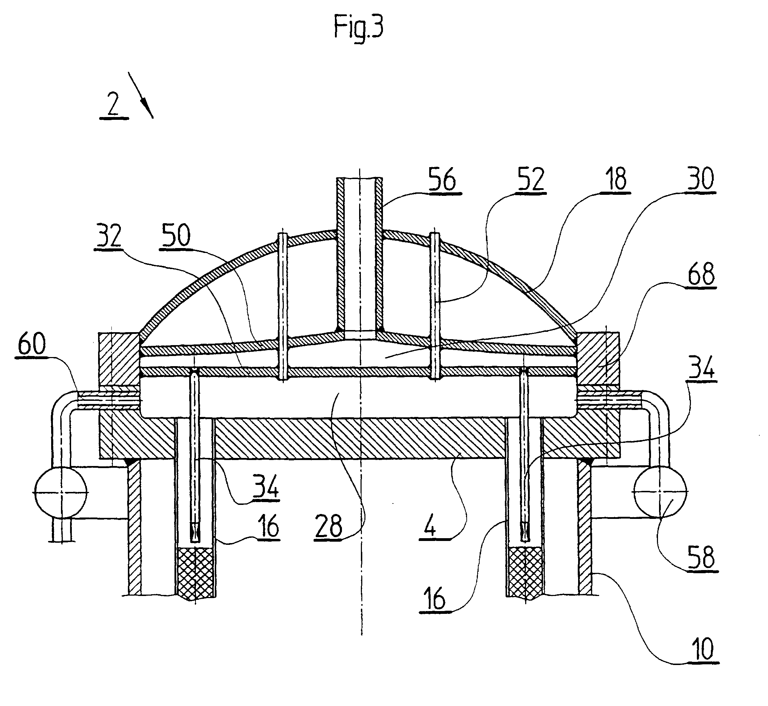

Die Ausführung gemäß Fig. 3 unterscheidet sich von derjenigen nach Fig. 2 insoweit, als hier die Haube 18 mit der Gaszuführungskammer 28 eine Einheit bildet, indem der Rohrboden 32 ebenso wie die Platte 50 an die Haube 18, genauer gesagt einen zylindrischen Flanschring 68 derselben, angeschweißt ist. In diesem Fall ist an den Tragstangen 52 neben der Platte 50 auch der Rohrboden 32 aufgehängt.

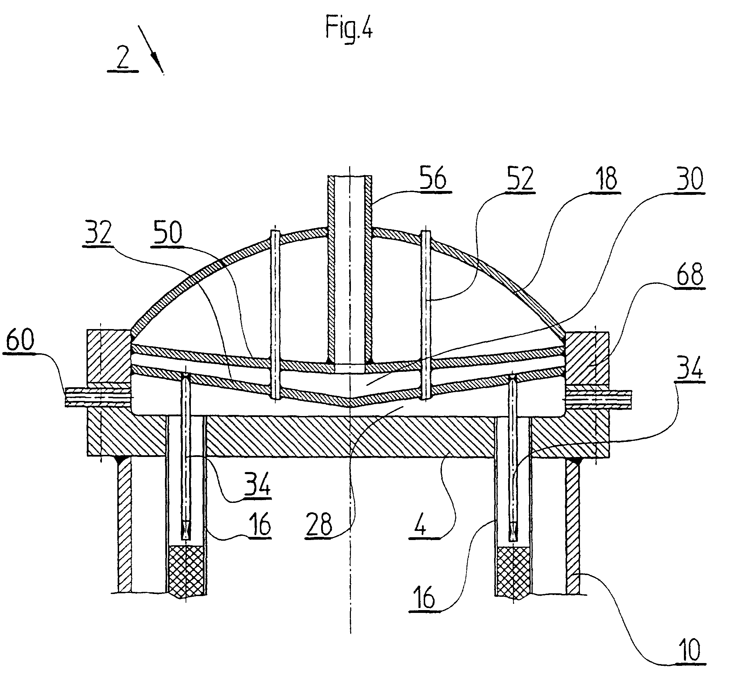

Nach Fig. 4 ist der Rohrboden 32 ebenso wie die Platte 50 konisch bzw. gewölbt, um aus den vorausgehend genannten Gründen neben der zweiten Gaszuführungskammer 30 auch der ersten Gaszuführungskammer 28 ein geringstmögliches Volumen und überdies dem Rohrboden 32 größere Steifigkeit zu geben. Davon abgesehen entspricht diese Ausführungsform weitgehend derjenigen aus Fig. 3.

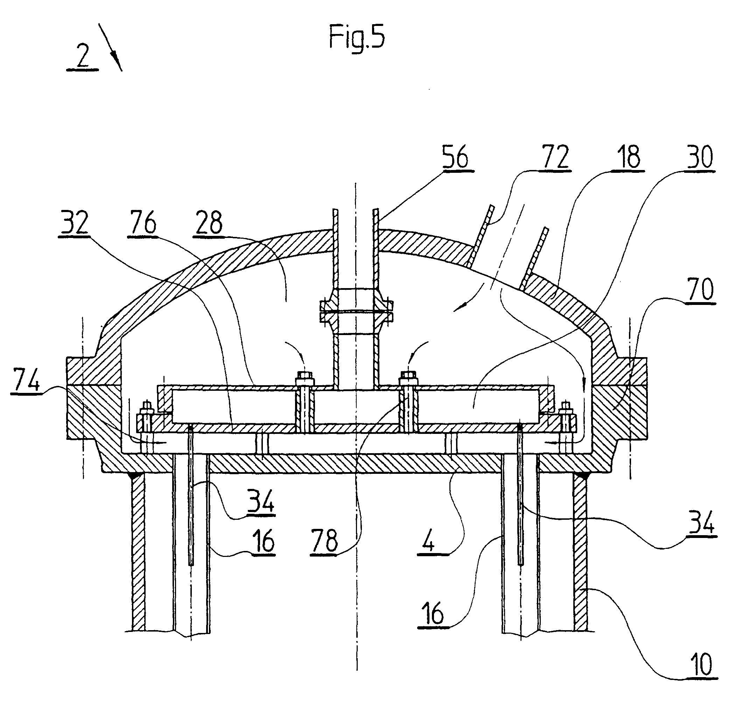

Nach Fig. 5 ist eine baulich separate zweite Gaszuführungskammer 30 innerhalb der ersten Gaszuführungskammer 28 angeordnet, die in diesem Fall von einem an die Peripherie des Rohrbodens 4 anschließenden zylindrischen Flanschansatz 70 sowie der gaseintrittsseitigen Haube 18 gebildet wird. Die Zuführung des ersten Reaktanten zu der Gaszuführungskammer 28 erfolgt durch einen außermittig in der Haube 18 angeordneten Rohrstutzen 72, während die Zuführung des zweiten Reaktanten zu der Gaszuführungskammer 30 wiederum durch ein die Haube 18 durchsetzendes zentrales Rohr 56 geschieht, das in diesem Fall jedoch zwecks Demontierbarkeit der Kammer 30 geteilt ist.

Der Rohrboden 32 der Gaszuführungskammer 30 ist über Schraubbolzen 74 mit Abstand über dem Rohrboden 4 angebracht. Die obere Begrenzung der Kammer 30 wird von einer flachen Schale 76 gebildet, die über hohle Schraubbolzen 78 mit dem Rohrboden 32 verschraubt ist. Fur den durch den Rohrstutzen 72 eintretenden ersten Reaktanten ist die Kammer 30 umgehbar, und überdies vermag er durch die hohlen Schraubbolzen 78 hindurchzutreten, um so seinen Weg in die Reaktionsrohre 16 zu finden.

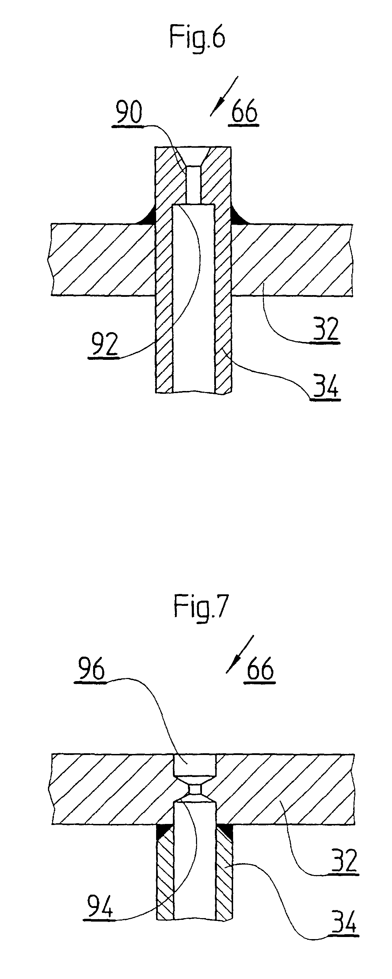

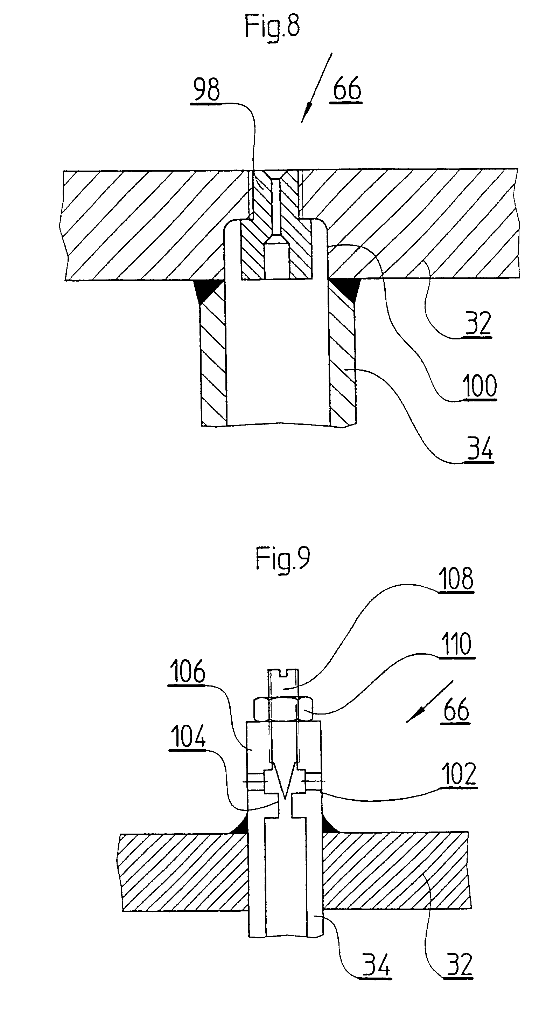

In den Figuren 6 - 9 ist jeweils der Rohrboden 32 der zweiten Gaszuführungskammer 30 mit einem darin ein- oder daran angeschweißten Gaszuführungsrohr 34 sowie die betreffende Drosselstelle 66 zu erkennen. Gemäß Fig. 6 wird die Drosselstelle 66 von einer angefasten Bohrung 90 innerhalb einer Stirnwand 92 des Rohres 34, gemäß Fig. 7 von einem Bund 94 innerhalb einer Durchbohrung 96 des Rohrbodens 32, gemäß Fig. 8 von einem hohlen, entsprechend dimensionierten Schraubnippel 98 innerhalb einer abgesetzten Durchbohrung 100 des Rohrbodens 32 und gemäß Fig. 9 von einer durch eine Kreuzbohrung 102 seitlich zugängigen axialen Bohrung 104 innerhalb eines massiven Endabschnitts 106 des betreffenden Rohres 34 in Verbindung mit einer axial in bezug auf die Bohrung 104 verstellbaren Stiftschraube 108 gebildet, die in ihrer jeweiligen Stellung durch eine Kontermutter 110 fixierbar ist.

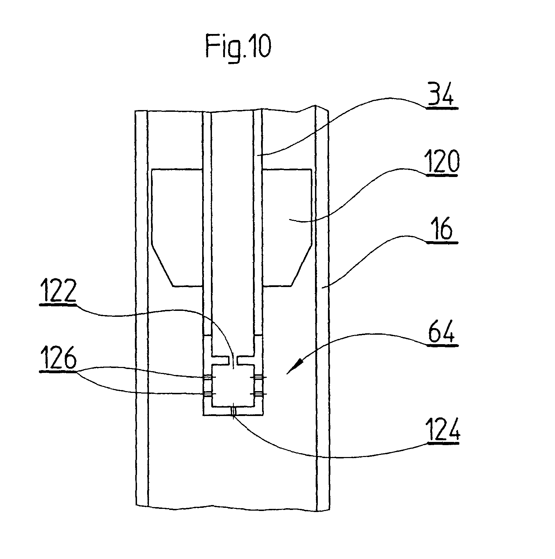

In Fig. 10 ist das stromabwärts gelegene Ende eines Gaszuführungsrohres 34 innerhalb des umgebenden Reaktionsrohres 16 zu erkennen. Wie ersichtlich wird dieses Ende in bezug auf das Rohr 16 durch Zentriermittel in Gestalt daran angebrachter Flügel 120 zentriert, die zwecks Erleichterung der Einführung in das Rohr 16 stirnseitig angefast sind. In bezug auf die in Fig. 1 dargestellte Zentrierplatte 46 können die Flügel 120 zugleich die Vorsprünge bilden, auf denen die Zentrierplatte bei Entfernung des Rohrbodens 32 mit den Gaszuführungsrohren 16 zur Auflage kommt.

Des weiteren zeigt die Figur eine Mischdüse 64 am Ende des Gaszuführungsrohres 34, die mit einer Drosselstelle 122 ähnlich dem vorausgehend beschriebenen Drosselorgan 66 am Anfang des Gaszuführungsrohres 34 kombiniert ist, welche die gleiche Funktion erfüllt. Im übrigen weist die hier gezeigte Mischdüse 64 neben einer stirnseitigen Gasaustrittsöffnung 124 mehrere teils hintereinanderliegende, teils diametral einander gegenüberliegende - oder auch kranzförmig verteilte - seitliche Gasaustrittsöffnungen 126 auf. Bei am Anfang des Gaszuführungsrohres angeordneter Drosselstelle, wie z.B. nach den Figuren 6 bis 9, könnten derartige seitliche Gasaustrittsstellen sich auch weiter zum Rohranfang hin und selbst bis außerhalb des jeweiligen Reaktionsrohres 16 fortsetzen, um so eine fortschreitende und möglichst intensive Beimischung des zweiten Reaktanten zu erreichen.

Um das Mischungsverhältnis der Reaktanten in bezug auf die Gesamtheit der Reaktionsrohre 16 noch weiter zu vergleichmäßigen, als dies etwa vermittels der Drosselorgane an den Gaszuführungsrohren möglich ist, kann dem ersten Reaktanten, z.B. Ethylen, in herkömmlicher Weise bereits vor Eintritt in den Reaktor 2 eine Teilmenge des zweiten Reaktanten, z.B. O₂, bis zu einer Größe zugemischt werden, welche für sich noch keine riskante Mischung entstehen läßt. Damit nämlich läßt sich die durch die Gaszuführungsrohre 34 im Reaktor 2 zugeführte Teilmenge des zweiten Reaktanten entsprechend reduzieren.

Es sei angemerkt, daß in bezug auf das angegebene Beispiel O₂ nicht notwendigerweise den durch die Gaszuführungsrohre zugegebenen zweiten Reaktanten bilden muß. Vielmehr ist es auch denkbar, O₂ - oder ein unkritisches Ethylen-O₂-Gemisch - als ersten Reaktanten über die erste Gaszuführungskammer 28 und Ethylen als zweiten Reaktanten über die zweite Gaszuführungskammer hindurch zuzuführen.

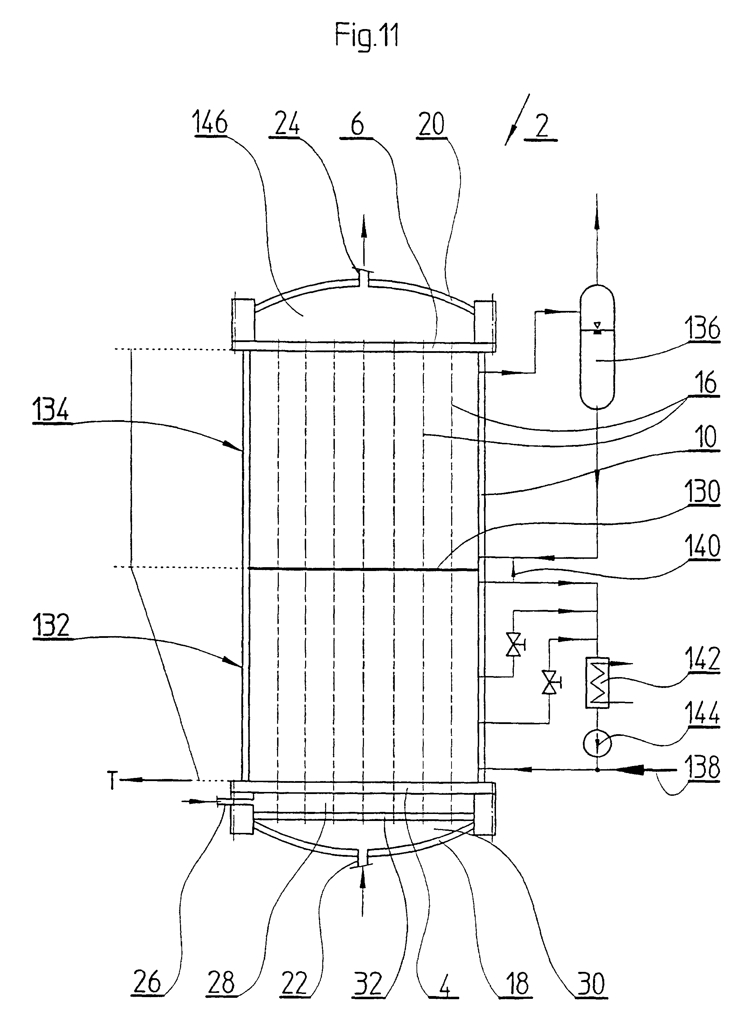

Fig. 11 zeigt, ebenso schematisch, einen im wesentlichen mit demjenigen nach Fig. 1 übereinstimmenden Rohrenreaktor 2, der allerdings innerhalb des Reaktormantels 10 durch eine transversale Trennplatte 130 in zwei in bezug auf den Wärmeträgerkreislauf unterschiedliche Bereiche, 132 und 134, unterteilt ist. Derartige Maßnahmen sind, wie gesagt, der eingangs genannten Veröffentlichung DE-C-2 201 528 (Fig. 5) zu entnehmen.

Links neben dem gezeigten Reaktor 2 ist diagrammäßig der Temperaturverlauf innerhalb des Reaktors entlang den Reaktionsrohren 16 dargestellt.

Während man mittels in dem Abschnitt 134 herbeigeführter Verdampfung des Wärmeträgers die Temperatur auf konstantem Niveau hält, läßt man in dem stromaufwärts gelegenen Abschnitt 132 die Temperatur des dort allein flüssigen Warmeträgers von dem gaseintrittsseitigen Rohrboden 4 hinweg stetig ansteigen. Auf diese Weise gelingt es, den Reaktor ohne Entzündungs- oder Explosionsgefahr und selbst ohne Gefahr einer lokalen Überhitzung, die an der nämlichen Stelle sogleich zu einer unerwünschten Endreaktion führen könnte, unter noch größerer Beladung des Reaktionsgasgemischs mit der kritischen Komponente, wie zum Beispiel O₂, zu fahren, als dies allein mit der verzögerten Zufuhrung über die aus der sekundären Gaszuführungskammer 30 gespeisten Gaszuführungsrohre 34 möglich wäre.

Selbstverständlich lassen sich mit entsprechender Auslegung des Wärmeträgerkreislaufs, ggf. in Verbindung mit mehreren Trennplatten wie der in Fig. 11 gezeigten Trennplatte 130, gewunschtenfalls auch noch kompliziertere Temperaturprofile entlang den Reaktionsrohren 16 erreichen. Zumeist kann man annehmen, daß die Reaktivität am Beginn der Reaktionsrohre infolge des hohen O₂-Anteils am höchsten ist, so daß es schon aus diesem Grunde wünschenswert ist, dort eine vergleichsweise niedere Temperatur zu haben, Im weiteren Verlauf der Reaktion nimmt die Reaktivität ab, was durch Erhöhung der Wärmetragertemperatur ausgeglichen werden kann. Ab einem bestimmten Umsetzungsgrad hingegen erscheint eine weitere Temperaturerhöhung unangebracht. In diesem Bereich also kann mit Verdampfung gearbeitet werden.

Der anfallende Dampf wird in einem Separator 136 von der flüssigen Phase getrennt und einer anderweitigen Verwendung zugefuhrt, während der flüssige Warmeträger in den Kreislauf zuruckgeführt wird. Andererseits wird, bei 138, der als Dampf abgeführte Warmeträger laufend ersetzt. Dazu stehen die Kreisläufe der Reaktorabschnitte 132 und 134 bei 140 miteinander in Verbindung. 142 ist ein Kühler und 144 eine Pumpe im Kreislauf des Abschnitts 132.

Sofern für beide Bereiche, 132 und 134, der gleiche Wärmeträger Verwendung findet und umsomehr, wenn ohnehin, wie in Fig. 11 gezeigt, die Kreisläufe beider Abschnitte miteinander in Verbindung stehen, braucht im übrigen die Trennplatte 130 nicht vollkommen dicht zu sein.

Unter Umständen kann das Reaktionsgas noch in der Gasauslaßkammer 146 unter der Haube 20 reaktionsfähig sein und so zu einem Brand führen. In solchen Fällen empfiehlt es sich, das Reaktionsgas noch vor Austritt aus den Reaktionsrohren 16 vermittels des Wärmeträgers zu kuhlen und überdies, etwa durch Einbauten, auch der Gasauslaßkammer 146 ein geringstmögliches Volumen zu geben, um so die Verweilzeit des austretenden Reaktionsgases darin zu verkürzen.

In bestimmten Fällen kann es auch noch wünschenswert sein, um so die Ausbeute pro Durchlauf noch weiter zu verbessern, bezuglich des hauptsächlichen Reaktionsgasstromes einen erfindungsgemäßen Röhrenreaktor mit einem oder mehreren gleich- oder auch andersartigen Reaktoren hintereinanderzuschalten.

## Patentansprüche

1. Röhrenreaktor (2) zur Durchführung exothermer Gasphasenreaktionen, mit einem sich abgedichtet zwischen zwei Rohrböden (4, 6) erstreckenden, von einem Reaktionsgasgemisch durchströmten und eine Katalysatorfüllung aufweisenden Reaktionsrohrbündel (8), das innerhalb eines umgebenden Reaktormantels (10) von einem Wärmeträger umspült wird, mit einer den jeweiligen Rohrboden (4, 6) überspannenden Haube (18, 20) in Verbindung mit einer Gaszuführungs- bzw. einer Gasabführungsleitung und mit gaseintrittsseitig in die Reaktionsrohre (16) hineinragenden und aus einer eigenen Gaszuführungsleitung speisbaren separaten Gaszuführungsrohren (34) für einen zweiten Reaktanten, **dadurch gekennzeichnet, daß** sich innerhalb der gaseintrittsseitigen Haube (18) neben einer mit dem Inneren der Reaktionsrohre (16) in Verbindung stehenden ersten Gaszuführungskammer (28) eine separat mit dem zweiten Reaktanten speisbare und einen eigenen Rohrboden (32) aufweisende zweite Gaszuführungskammer (30) in Verbindung mit den separaten separaten Gaszuführungsrohren (34) befindet, aus der diese separaten Gaszuführungsrohre über jeweils ein eigenes Drosselorgan (66; 122) speisbar sind.

2. Röhrenreaktor (2) nach Anspruch 1, **dadurch gekennzeichnet, daß** zumindest eine der beiden Gaszuführungskammern (28, 30) ein wesentlich geringeres Volumen besitzt als der verfügbare Raum unter der gaseintrittsseitigen Haube (18) dies zulassen würde.

3. Röhrenreaktor (2) nach Anspruch 2, **dadurch gekennzeichnet, daß** die betreffende Gaszuführungskammer (28, 30) gegenüber dem verfügbaren Volumen durch mindestens einen Einbau (50) verkleinert ist.

4. Röhrenreaktor (2) nach Anspruch 3, **dadurch gekennzeichnet, daß** der Einbau aus einer in die gaseintrittsseitige Haube (18) transversal verlaufend eingeschweißten Platte (50) besteht.

5. Röhrenreaktor (2) nach Anspruch 4, **dadurch gekennzeichnet, daß** die Platte (50) zusätzlich in ihrem mittleren Bereich an der gaseintrittsseitigen Haube (18) abgestützt ist.

6. Röhrenreaktor (2) nach Anspruch 5, **dadurch gekennzeichnet, daß** die Abstützung zumindest teilweise von einem entsprechend massiven zentralen Gaszuführungsrohr (56) gebildet wird.

7. Röhrenreaktor (2) nach einem der Ansprüche 4 bis 6, **dadurch gekennzeichnet, daß** die Platte (50) und ggf. ebenso der Rohrboden (32) der zweiten Gaszuführungskammer (30) im wesentlichen gewölbt oder konisch ist.

8. Röhrenreaktor (2) nach einem der vorgehenden Ansprüche, **dadurch gekennzeichnet, daß** der Rohrboden (32) der zweiten Gaszuführungskammer (30) gegenüber dem gaseintrittsseitigen Rohrboden (4) des Reaktionsrohrbündels (8) und/oder gegenüber der gaseintrittsseitigen Haube (18) abgestützt ist.

9. Röhrenreaktor (2) nach einem der vorgehenden Ansprüche, **dadurch gekennzeichnet, daß** der Rohrboden (32) der zweiten Gaszuführungskammer (30) zwischen einem Flansch (40) der gaseintrittsseitigen Haube (18) und einem solchen (36) in Verbindung mit dem Reaktormantel (10) eingespannt ist.

10. Röhrenreaktor (2) nach einem der Ansprüche 1 bis 8, **dadurch gekennzeichnet, daß** der Rohrboden (32) der zweiten Gaszuführungskammer (30) samt dieser lösbar an dem gaseintrittsseitigen Rohrboden (4) des Reaktionsrohrbündels (8) angebracht ist.

11. Röhrenreaktor (2) nach einem der Ansprüche 1 bis 8, **dadurch gekennzeichnet, daß** die gesamte zweite Gaszuführungskammer (30) mit der gaseintrittsseitigen Haube (18) integriert ist.

12. Röhrenreaktor (2) nach einem der vorgehenden Ansprüche, **dadurch gekennzeichnet, daß** die zweite Gaszuführungskammer (30) samt den daran anschließenden separaten Gaszuführungsrohren (34) ebenso wie die gaseintrittsseitige Haube (18) abnehmbar ist.

13. Röhrenreaktor (2) nach einem der vorgehenden Ansprüche, **dadurch gekennzeichnet, daß** zumindest eine der beiden Gaszuführungskammern (28, 30) eine Gasverteilungsplatte (41) mit Durchbrechungen variierenden Querschnitts aufweist.

14. Röhrenreaktor (2) nach einem der vorgehenden Ansprüche, **dadurch gekennzeichnet, daß** zumindest eine der Gaszuführungskammern (28, 30) von der Reaktorlängsmittelachse hinweg eine nach Strömungsverteilungsgesichtspunkten zu bzw. abnehmende Höhe aufweist.

15. Röhrenreaktor (2) nach einem der vorgehenden Ansprüche, **dadurch gekennzeichnet, daß** die separaten Gaszuführungsrohre (34) vor Eintritt in die Reaktionsrohre (16) durch eine vorzugsweise gasdurchlässige - Zentrierplatte (46) hindurchgeführt sind.

16. Röhrenreaktor (2) nach Anspruch 15, **dadurch gekennzeichnet, daß** die Zentrierplatte (46) auf den separaten Gaszuführungsrohren (34) prinzipiell bis zu deren freien Enden hin verschiebbar ist.

17. Röhrenreaktor (2) nach einem der vorgehenden Ansprüche, **dadurch gekennzeichnet, daß** die separaten Gaszuführungsrohre (34) an ihren freien Enden Zentriermittel (120) zu ihrer Zentrierung in bezug auf die zugehörigen Reaktionsrohre (16) aufweisen.

18. Röhrenreaktor (2) nach einem der vorgehenden Ansprüche, **dadurch gekennzeichnet, daß** die separaten Gaszuführungsrohre (34) zumindest an ihren freien Enden Mischdüsen (64) zur Verwirbelung des durch sie zugeführten zweiten Reaktanten mit dem ersten aufweisen.

19. Röhrenreaktor (2) nach einem der vorgehenden Ansprüche, **dadurch gekennzeichnet, daß** die Drosselorgane (66) der separaten Gaszuführungsrohre (34) individuell einstellbar sind.

20. Röhrenreaktor (2) nach Anspruch 18 oder 19, **dadurch gekennzeichnet, daß** die Drosselorgane (122) mit den Mischdüsen (64) jeweils eine Einheit bilden.

21. Röhrenreaktor (2) nach einem der vorhergehenden Ansprüche, **dadurch gekennzeichnet, daß** die separaten Gaszuführungsrohre (34) jeweils mehrere über ihre Länge verteilte Gasaustrittsstellen aufweisen.

22. Röhrenreaktor (2) nach Anspruch 21, **dadurch gekennzeichnet, daß** die betreffenden Gasaustrittsstellen zum Teil noch außerhalb der Reaktionsrohre (16) liegen.

23. Röhrenreaktor (2) nach einem der vorgehenden Ansprüche, **dadurch gekennzeichnet, daß** die separaten Gaszuführungsrohre (34) noch vor der in dem jeweiligen Reaktionsrohr (16) enthaltenen Katalysatorfüllung (62) enden.

24. Röhrenreaktor (2) nach Anspruch 23, **dadurch gekennzeichnet, daß** die separaten Gaszuführungsrohre (34) in einer der Katalysatorfüllung (62) vorgelagerten Inertmaterialschicht enden.

25. Röhrenreaktor (2) nach einem der vorgehenden Ansprüche, **dadurch gekennzeichnet, daß** der Wärmeträgerkreislauf eine Einrichtung aufweist, die die Betriebstemperatur von einem verhältnismäßig niedrigen Wert am Gaseintrittsende der Reaktionsrohre (16) hinweg entlang den Reaktionsrohren steigert.

26. Röhrenreaktor (2) nach Anspruch 25, **dadurch gekennzeichnet, daß** der Wärmeträgerkreislauf des weiteren eine Einrichtung aufweist, die die Betriebstemperatur in einem weiter stromabwärts gelegenen Abschnitt der Reaktionsrohre (16) konstant hält.

27. Röhrenreaktor (2) nach Anspruch 26, **dadurch gekennzeichnet, daß** im Bereich des betreffenden Rohrabschnitts eine Einrichtung zur Verdampfung des Wärmeträgers vorhanden ist.

28. Röhrenreaktor (2) nach einem der vorhergehenden Ansprüche, **dadurch gekennzeichnet, daß** der Wärmeträgerkreislauf eine Einrichtung aufweist, die die Betriebstemperatur zum Gasaustrittsende der Reaktionsrohre (16) hin abzusenkt.

29. Röhrenreaktor (2) nach einem der vorgehenden Ansprüche,
**dadurch gekennzeichnet, daß** eine Gasauslaßkammer (146) innerhalb der gasaustrittsseitigen Haube (20) durch Einbauten oder dergl. ein geringstmögliches Volumen besitzt.

30. Röhrenreaktor (2) nach einem der vorhergehenden Ansprüche, **dadurch gekennzeichnet, daß** eine Einrichtung vorhanden ist, die einen Teil des durch die separaten Gaszuführungsrohre (34) in die Reaktionsrohre eingespeisten zweiten Reaktanten dem ersten Reaktanten bereits vor Eintritt in den Reaktor zumischt.

31. Röhrenreaktor (2) nach einem der vorhergehenden Ansprüche, **dadurch gekennzeichnet, daß** er in bezug auf den hauptsächlichen Reaktionsgasstrom mit einem oder mehreren gleichoder andersartigen Reaktoren in Reihe geschaltet ist.

## Claims

1. Tubular reactor (2) for carrying out exothermic gas phase reactions, comprising a reaction tube bundle (8) which extends in a sealed manner between two tube bottoms (4, 6), is flowed through by a reaction gas mixture, has a catalyst filling and within a surrounding reactor jacket (10) is passed around by a heat transfer medium, and comprising a hood (18, 20) which covers the respective tube bottom (4, 6) and is connected to a gas supply and gas discharge line, and comprising separate gas supply tubes (34) for a second reactant which project into the reaction tubes (16) on the gas inlet side and can be fed from a dedicated gas supply line, **characterized in that** within the gas-inlet-side hood (18) there is, besides a first gas supply chamber (28) that is connected to the interior of the reaction tubes (16), a second gas supply chamber (30) which can be fed separately with the second reactant, has a dedicated tube bottom (32) and is connected to the separate gas supply tubes (34), from which second gas supply chamber these separate gas supply tubes can be fed via in each case one dedicated flow control member (66; 122).

2. Tubular reactor (2) according to Claim 1, **characterized in that** at least one of the two gas supply chambers (28, 30) has a significantly smaller volume than the available space under the gas-inlet-side hood (18) would allow.

3. Tubular reactor (2) according to Claim 2, **characterized in that** the relevant gas supply chamber (28, 30) is made smaller than the available volume by means of at least one built-in component (50).

4. Tubular reactor (2) according to Claim 3, **characterized in that** the built-in component consists of a plate (50) that is welded into the gas-inlet-side hood (18) in a transversely running manner.

5. Tubular reactor (2) according to Claim 4, **characterized in that** the plate (50) is additionally supported in its middle region on the gas-inlet-side hood (18).

6. Tubular reactor (2) according to Claim 5, **characterized in that** the support is formed at least partially by an appropriately sturdy central gas supply tube (56).

7. Tubular reactor (2) according to any of Claims 4 to 6, **characterized in that** the plate (50), and possibly also the tube bottom (32) of the second gas supply chamber (30), is essentially curved or conical.

8. Tubular reactor (2) according to any of the preceding claims, **characterized in that** the tube bottom (32) of the second gas supply chamber (30) is supported with respect to the gas-inlet-side tube bottom (4) of the reaction tube bundle (8) and/or with respect to the gas-inlet-side hood (18).

9. Tubular reactor (2) according to any of the preceding claims, **characterized in that** the tube bottom (32) of the second gas supply chamber (30) is mounted between a flange (40) of the gas-inlet-side hood (18) and a flange (36) that is connected to the reactor jacket (10).

10. Tubular reactor (2) according to any of Claims 1 to 8, **characterized in that** the tube bottom (32) of the second gas supply chamber (30), together with the latter, is releasably attached on the gas-inlet-side tube bottom (4) of the reaction tube bundle (8).

11. Tubular reactor (2) according to any of Claims 1 to 8, **characterized in that** the entire second gas supply chamber (30) is integrated with the gas-inlet-side hood (18).

12. Tubular reactor (2) according to any of the preceding claims, **characterized in that** the second gas supply chamber (30), together with the separate gas supply tubes (34) connected thereto and like the gas-inlet-side hood (18), can be removed.

13. Tubular reactor (2) according to any of the preceding claims, **characterized in that** at least one of the two gas supply chambers (28, 30) has a gas distribution plate (41) with openings of varying cross section.

14. Tubular reactor (2) according to any of the preceding claims, **characterized in that** at least one of the gas supply chambers (28, 30) has a height that increases and decreases, with regard to the flow distribution, in the direction away from the longitudinal centre axis of the reactor.

15. Tubular reactor (2) according to any of the preceding claims, **characterized in that** the separate gas supply tubes (34) are guided through a - preferably gas-permeable - centring plate (46) before entering the reaction tubes (16).

16. Tubular reactor (2) according to Claim 15, **characterized in that** the centring plate (46) can be displaced on the separate gas supply tubes (34) in principle up to the free ends thereof.

17. Tubular reactor (2) according to any of the preceding claims, **characterized in that** the separate gas supply tubes (34) have, at their free ends, centring means (120) for centring them with respect to the associated reaction tubes (16).

18. Tubular reactor (2) according to any of the preceding claims, **characterized in that** the separate gas supply tubes (34) have, at least at their free ends, mixing nozzles (64) for swirling the second reactant, fed by said separate gas supply tubes, with the first reactant.

19. Tubular reactor (2) according to any of the preceding claims, **characterized in that** the flow control members (66) of the separate gas supply tubes (34) can be adjusted individually.

20. Tubular reactor (2) according to Claim 18 or 19, **characterized in that** the flow control members (122) in each case form a unit with the mixing nozzles (64).

21. Tubular reactor (2) according to any of the preceding claims, **characterized in that** the separate gas supply tubes (34) in each case have a number of gas outlet points distributed over their length.

22. Tubular reactor (2) according to Claim 21, **characterized in that** some of the relevant gas outlet points still lie outside the reaction tubes (16).

23. Tubular reactor (2) according to any of the preceding claims, **characterized in that** the separate gas supply tubes (34) still end before the catalyst filling (62) contained in the respective reaction tube (16).

24. Tubular reactor (2) according to Claim 23, **characterized in that** the separate gas supply tubes (34) end in an inert material layer placed in front of the catalyst filling (62).

25. Tubular reactor (2) according to any of the preceding claims, **characterized in that** the heat transfer medium cycle has a device which raises the operating temperature along the reaction tubes from a relatively low value at the gas inlet end of the reaction tubes (16).

26. Tubular reactor (2) according to Claim 25, **characterized in that** the heat transfer medium cycle furthermore has a device which keeps the operating temperature constant in another section of the reaction tubes (16) that is located downstream.

27. Tubular reactor (2) according to Claim 26, **characterized in that** in the region of the relevant tube section there is a device for evaporating the heat transfer medium.

28. Tubular reactor (2) according to any of the preceding claims, **characterized in that** the heat transfer medium cycle has a device which lowers the operating temperature towards the gas outlet end of the reaction tubes (16).

29. Tubular reactor (2) according to any of the preceding claims, **characterized in that** a gas outlet chamber (146) within the gas-outlet-side hood (20) has a volume that is as low as possible, by virtue of built-in components or the like.

30. Tubular reactor (2) according to any of the preceding claims, **characterized in that** there is a device which mixes part of the second reactant, fed into the reaction tubes by the separate gas supply tubes (34), with the first reactant prior to entry into the reactor.

31. Tubular reactor (2) according to any of the preceding claims, **characterized in that**, with respect to the main flow of reaction gas, it is connected in series with one or more identical or different reactors.

## Revendications

1. Réacteur tubulaire (2) pour l'exécution de réactions exothermiques en phase gazeuse, comprenant un faisceau de tubes de réaction (8) s'étendant entre deux fonds tubulaires (4, 6), parcouru par un mélange de gaz de réaction et présentant une charge de catalyseur, faisceau qui est léché par un fluide caloporteur à l'intérieur d'une enceinte de réacteur (10) qui l'entoure, un dôme (18, 20) qui recouvre chaque fond tubulaire (4, 6), en communication avec une conduite d'amenée de gaz ou une conduite d'évacuation de gaz, et des tubes d'amenée de gaz séparés (34) pour un deuxième réactif, qui sont engagés dans les tubes de réaction (16) sur le côté entrée de gaz et qui peuvent être alimentés à partir d'une conduite d'amenée de gaz qui leur est propre, **caractérisé en ce que**, à l'intérieur du dôme côté entrée de gaz (18), se trouve, en supplément d'une première chambre d'amenée de gaz (28) qui est en communication avec l'intérieur des tubes de réaction (16), une deuxième chambre d'amenée de gaz (30) pouvant être alimentée séparément avec le deuxième réactif et qui présente un fond tubulaire (32) qui lui est propre, chambre par laquelle ces tubes d'amenée de gaz séparés (34) peuvent être alimentés chacun par l'intermédiaire d'un organe d'étranglement (66 ; 122) qui lui est propre.

2. Réacteur tubulaire (2) selon la revendication 1, **caractérisé en ce qu'**au moins une des deux chambres d'amenée de gaz (28, 30) possède un volume considérablement inférieur à celui qu'admettrait l'espace disponible sous le dôme côté entrée de gaz (18).

3. Réacteur tubulaire (2) selon la revendication 2, **caractérisé en ce que** la chambre d'amenée de gaz (28, 30) concernée est réduite relativement au volume disponible par au moins un élément intérieur (50).

4. Réacteur tubulaire (2) selon la revendication 3, **caractérisé en ce que** l'élément intérieur est constitué par une plaque soudée (50) qui s'étend transversalement dans le dôme côté entrée de gaz (18).

5. Réacteur tubulaire (2) selon la revendication 4, **caractérisé en ce que** la plaque (50) est en supplément appuyée sur le dôme côté entrée de gaz (18) dans sa région centrale.

6. Réacteur tubulaire (2) selon la revendication 5, **caractérisé en ce que** l'appui est au moins partiellement formé par un tube d'amenée de gaz central (56) d'une masse appropriée.

7. Réacteur tubulaire (2) selon une des revendications 4 à 6, **caractérisé en ce que** la plaque (50) et éventuellement aussi le fond tubulaire (32) de la deuxième chambre d'amenée de gaz (30) sont sensiblement bombés ou coniques.

8. Réacteur tubulaire (2) selon une des revendications précédentes, **caractérisé en ce que** le fond tubulaire (32) de la deuxième chambre d'amenée de gaz (30) prend appui par rapport au fond tubulaire côté entrée de gaz (4) du faisceau de tubes de réaction (8) et/ou par rapport au dôme côté entrée de gaz (18).

9. Réacteur tubulaire (2) selon une des revendications précédentes, **caractérisé en ce que** le fond tubulaire (32) de la deuxième chambre d'amenée de gaz (30) est encastré entre une bride (40) du dôme côté entrée de gaz (18) et une bride (36) qui est reliée à l'enceinte (10) du réacteur.

10. Réacteur tubulaire (2) selon une des revendications 1 à 8, **caractérisé en ce que** le fond tubulaire (32) de la deuxième chambre d'amenée de gaz (30) est monté, avec cette chambre, de façon démontable, sur le fond tubulaire côté entrée de gaz (4) du faisceau de tubes de réaction (8).

11. Réacteur tubulaire (2) selon une des revendications 1 à 8, **caractérisé en ce que** la totalité de la deuxième chambre d'amenée de gaz (30) est intégrée au dôme côté entrée de gaz (18).

12. Réacteur tubulaire (2) selon une des revendications précédentes, **caractérisé en ce que** la deuxième chambre d'amenée de gaz (30) peut être enlevée, avec les tubes d'amenée de gaz séparés (34) qui y sont raccordés, de même que le dôme côté entrée de gaz (18).

13. Réacteur tubulaire (2) selon une des revendications précédentes, **caractérisé en ce qu'**au moins une des deux chambres d'amenée de gaz (28, 30) présente une plaque de distribution du gaz (41) présentant des ajours de section variable.

14. Réacteur tubulaire (2) selon une des revendications précédentes, **caractérisé en ce qu'**au moins une des chambres d'amenée de gaz (28, 30) présente une hauteur qui croît ou décroît à partir de l'axe médian longitudinal du réacteur, selon les besoins de la distribution de l'écoulement.

15. Réacteur tubulaire (2) selon une des revendications précédentes, **caractérisé en ce que** les tubes d'amenée de gaz séparés (34) sont enfilés à travers une plaque de centrage (46) - de préférence perméable aux gaz - avant d'entrer dans les tubes de réaction (16)

16. Réacteur tubulaire (2) selon la revendication 15, **caractérisé en ce que** la plaque de centrage (46) peut coulisser sur les tubes d'amenée de gaz séparés (34), en principe jusqu'à leurs extrémités libres.

17. Réacteur tubulaire (2) selon une des revendications précédentes, **caractérisé en ce que** les tubes d'amenée de gaz séparés (34) présentent à leurs extrémités libres des moyens de centrage (120) destinés à les centrer par rapport aux tubes de réaction correspondants (16).

18. Réacteur tubulaire (2) selon une des revendications précédentes, **caractérisé en ce que** les tubes d'amenée de gaz séparés (34) présentent au moins à leurs extrémités libres des buses de mélange (64) destinées à mettre le deuxième réactif amené par ces tubes dans un état turbulent avec le premier.

19. Réacteur tubulaire (2) selon une des revendications précédentes, **caractérisé en ce que** les organes d'étranglement (66) des tubes d'amenée de gaz séparés (34) peuvent être réglés individuellement.

20. Réacteur tubulaire (2) selon la revendication 18 ou 19, **caractérisé en ce que** les organes d'étranglement (122) forment chacun une unité avec les buses de mélange (64).

21. Réacteur tubulaire (2) selon une des revendications précédentes, **caractérisé en ce que** les tubes d'amenée de gaz séparés (34) présentent chacun plusieurs zones de sortie de gaz réparties sur leur longueur.

22. Réacteur tubulaire (2) selon la revendication 21, **caractérisé en ce que** les zones de sortie de gaz en question se trouvent en partie encore en dehors des tubes de réaction (16).

23. Réacteur tubulaire (2) selon une des revendications précédentes, **caractérisé en ce que** les tubes d'amenée de gaz séparés (34) se terminent chacun avant d'atteindre la charge de catalyseur (62) contenue dans le tube de réaction (16) respectif.

24. Réacteur tubulaire (2) selon la revendication 23, **caractérisé en ce que** les tubes d'amenée de gaz séparés (34) se terminent dans une couche de matière inerte placée en amont de la charge de catalyseur (62).

25. Réacteur tubulaire (2) selon une des revendications précédentes, **caractérisé en ce que** le circuit de fluide caloporteur présente un dispositif qui fait croître la température de fonctionnement à partir d'une valeur relativement basse à l'extrémité d'entrée de gaz des tubes de réaction (16), sur la longueur de ces tubes de réaction.

26. Réacteur tubulaire (2) selon la revendication 25, **caractérisé en ce que** le circuit de fluide caloporteur présente par ailleurs un dispositif qui maintient la température de fonctionnement constante dans un segment des tubes de réaction (16) qui est placé plus loin en aval.

27. Réacteur tubulaire (2) selon la revendication 26, **caractérisé en ce que**, dans la région du segment de tube concerné, est présent un dispositif pour la vaporisation du fluide caloporteur.

28. Réacteur tubulaire (2) selon une des revendications précédentes, **caractérisé en ce que** le circuit de fluide caloporteur présente un dispositif qui abaisse la température de fonctionnement vers l'extrémité de sortie des tubes de réacteur (16).

29. Réacteur tubulaire (2) selon une des revendications précédentes, **caractérisé en ce qu'**une chambre de sortie de gaz (146) possède à l'intérieur du dôme côté sortie de gaz (20) un volume rendu aussi faible que possible par la présence d'élément intérieurs ou analogues.

30. Réacteur tubulaire (2) selon une des revendications précédentes, **caractérisé en ce qu'**il est prévu un dispositif qui mélange une partie du deuxième réactif introduit par les tubes d'amenée de gaz séparés (34) dans les tubes de réaction au premier réactif dès avant l'entrée dans le réacteur.

31. Réacteur tubulaire (2) selon une des revendications précédentes, **caractérisé en ce qu'**il est raccordé en série avec un ou plusieurs réacteurs du même type ou d'un autre type en ce qui concerne le courant de gaz de réaction principal.
